# EUROPÄISCHE PATENTSCHRIFT

(11) **EP 1 454 638 B1**
(45) Veröffentlichungstag und Bekanntmachung des Hinweises auf die Patenterteilung: **22.12.2010**
(21) Anmeldenummer: 04100769.1
(22) Anmeldetag: 26.02.2004
(51) Int. Cl.: A61L 2/18, A01N 33/12

(54) **Desinfektionsmittelkonzentrate auf Basis quaternärer Ammoniumverbindungen sowie die Verwendung derselben zur chemothermischen Instrumentenaufbereitung**
Concentrated disinfecting composition based on quaternary ammonium compounds and their use for chemical-thermal care for instruments
Désinfectants concentrés à base d'ammoniums quaternaires et leur utilisation pour la maintenance chimio-thermique d'instruments

(30) Priorität: 07.03.2003 DE 10310377
(43) Veröffentlichungstag der Anmeldung: 08.09.2004
(73) Patentinhaber: Bode Chemie GmbH, 22525 Hamburg (DE)
(72) Erfinder: Fehling, Thomas, 22305 Hamburg (DE); Knieler, Roland, Dr., 21227 Bendestorf (DE); Bloss, Richard, 25462 Rellingen (DE)
(74) Vertreter: Kossak, Sabine

(56) Entgegenhaltungen:
- EP-A- 0 940 145
- DE-A- 10 035 248
- US-A1- 2002 173 437
- US-A1- 2003 111 097

## Beschreibung

Die vorliegende Erfindung betrifft Desinfektionsmittelkonzentrate und ihre Verwendung zur maschinellen Instrumentendesinfektion.

Als Desinfektionsmittel zur Desinfektion von Instrumenten und Wäsche sind eine Vielzahl mikrobizid wirksamer chemischer Substanzen bzw. Gemische dieser Substanzen an sich bekannt. Mikrobizide Substanzen sind im allgemeinen gegen das übliche Spektrum von Keimen, wie beispielsweise grampositive Bakterien, gramnegative Bakterien, Mykobakterien, Hefen, Pilze, Viren und dergleichen, mehr oder weniger wirksam, so daß man üblicherweise eine ausreichende Desinfektion durch geeignete Wirkstoffkombinationen erzielen kann.

Der Stand der Technik kennt zur Desinfektion von medizinisch-chirurgischen Instrumenten eine Reihe von Wirkstoffen, insbesondere Aldehyde, wie beispielsweise Formaldehyd oder Glutaraldehyd, quaternäre Ammoniumverbindungen und langkettige Amine sowie Phenole. Allerdings weisen die genannten Verbindungen einige Nachteile auf:

Aldehyde fixieren Reste von Blut und Eiweiß durch chemische Reaktion an den zu desinfizierenden Gegenständen, so daß diese nach der Desinfektion schwer zu reinigen sind.

Quaternäre Ammoniumverbindungen und langkettige Amine werden häufig in der manuellen Instrumentendesinfektion verwendet. Im Vergleich zu den Aldehyden ist der Geruch dieser Verbindungen deutlich weniger unangenehm. Eine chemische Reaktion mit Eiweißen erfolgt nicht, jedoch kommt es zu einer physikalischen Fällung von Eiweißen, die zum Teil durch geschickte Kombination mit Tensiden kompensiert werden kann. Für die maschinelle Instrumentendesinfektion sind die quaternären Ammoniumverbindungen allerdings nicht geeignet, weil es infolge der Turbulenzen in der Reinigungsmaschine zu einer starken, unerwünschten Schaumbildung kommt. Ein weiterer entscheidender Nachteil ist das eingeengte Wirkungsspektrum quaternärer Ammoniumverbindungen, die nicht gegen gramnegative Bakterien wirken.

Auch Alkylamine, wie beispielsweise N,N-Bis-(3-aminopropyl)-laurylamin, sind prinzipiell zur Instrumentendesinfektion geeignet. Allerdings haben auch sie den Nachteil, in einer Reinigungsmaschine stark zu schäumen, weshalb auch sie sich für die chemo-thermische Desinfektion bislang nicht durchgesetzt haben.

Phenole sind vor allem wegen ihres Geruches, ihrer geringen Wirksamkeit gegen den Poliovirus, ihrer zum Teil schlechten Abbaubarkeit, ihrer hohen Lipidlöslichkeit verbunden mit einer starken Penetration durch die Haut sowie toxischer und mutagener Risiken in nahezu allen Anwendungsbereichen für Desinfektionsmittel auf dem Rückzug.

In der US 2002/0173437 A1 wird eine Methode zur Reinigung medizinischer Gerätschaften und Instrumente beschrieben, welche die Verwendung einer Reinigungszusammensetzung, einer Spülzusammensetzung und die Behandlung mit einer antimikrobiellen Zusammensetzung umfasst, wobei für die einzelnen Schritte unterschiedliche Zusammensetzungen offenbart sind. Bei jeder dieser Zusammensetzungen handelt es sich um feste Zusammensetzungen, welche in Wasser zu lösen sind. Jede dieser Zusammensetzungen soll nur einen begrenzten Zweck erfüllen, so dass auch entsprechend die Bestandteile gewählt sind. Die antimikrobielle Zusammensetzung beispielsweise umfasst quaternäre Ammoniumverbindungen oder feste Halogenverbindungen, beispielsweise chloriertes Trinatriumphosphat, als wesentliche Bestandteile.

In der EP 0 940 145 (entsprechend der DE 198 08 964) werden O/W-Mikroemulsionen mit einem oder mehreren Alkylthiouronium- und/oder α-ω-Alkylendithiouroniumsalzen als mikrobizide Wirkstoffe sowie die Verwendung derselben zur manuellen und/oder maschinellen Instrumentendesinfektion beansprucht.

In der DE 100 40 664 werden aldehydfreie, maschinelle Instrumentendesinfektionsmittel beansprucht, welche als Wirkstoffe quaternäre Ammoniumverbindungen und/oder Alkylamine und/oder Alkylthiouroniumverbindungen und/oder α,ω-Alkylendithiouroniumsalze enthalten. Zur Verhinderung der Schaumbildung enthalten diese Präparate mindestens einen hydrophoben Schaumregulator, wobei dieser bevorzugt N-Alkyl-isononansäureamid ist.

Instrumentendesinfektionsmittel nach den vorstehend genannten Schutzrechten bilden zwar in der Maschine nur wenig Schaum aus, jedoch zeigte sich bei häufigerer Anwendung, dass sich auf den Instrumenten Beläge bildeten. Diese Beläge lassen sich nur schwer entfernen und können zu Materialverträglichkeitsproblemen mit den empfindlichen thermolabilen Instrumenten und darüberhinaus zu einer Beeinträchtigung der mikrobiziden Wirksamkeit führen.

Auf der anderen Seite besteht aber ein grosser Bedarf an Desinfektionsmitteln für die chemo-thermische Instrumentendesinfektion, die einerseits die Vorteile aldehydfreier Wirkstoffe aufweisen, andererseits aber deren Nachteile nicht zeigen.

Aufgabe der vorliegenden Erfindung war es daher, Desinfektionsmittel zur maschinellen Instrumentendesinfektion zu finden, welche ohne Verwendung von Aldehyden die Nachteile des Standes der Technik nicht aufweisen, welche also eine gute Reinigungsleistung bei zugleich geringer Toxizität zeigen, weder Eiweiß noch Blut an den zu desinfizierenden Gegenständen fixieren, keinen unangenehmen Geruch haben und insbesondere in Wasch- und Desinfektionsautomaten für chirurgische Instrumente keine störende Schaumbildung und keine Belagbildung verursachen.

Es war überraschend und für den Fachmann nicht vorauszusehen, daß
Desinfektionsmittelformulierungen, welche neben einem Gehalt an mindestens einen Wirkstoff gewählt aus der Gruppe Benzalkoniumchlorid, N-Dodecyl-N,N-dimethyl-ethylbenzylammoniumchlorid, Dioctyldimethylammoniumchlorid, Didecyldimethylammoniumchlorid, Decylisononyldimethylammoniumchlorid und Didecylmethylpolyoxyethylammoniumpropionat
a) 1 bis 50 Gew.-% mindestens einer Komplexbildnerkomponente,
b) 1 bis 50 Gew.-% mindestens eines Lösungsvermittlers,
c) 1 bis 50 Gew.-% mindestens einer Hydroxycarbonsäure und
d) 0,1 bis 5 Gew.-% mindestens eines Alkansäure-Alkylesters als Schaumregulatorkomponente
enthalten,
wobei die Prozentangaben jeweils auf das Gesamtgewicht der Formulierungen bezogen sind,
den geschilderten Nachteilen des Standes der Technik Abhilfe schaffen würden.

Desinfektionsmittelformulierungen im Sinne der vorliegenden Erfindung sind besonders geeignet zum Desinfizieren von Instrumenten, insbesondere zur chemothermischen maschinellen Instrumentenaufbereitung bei 50 bis 60 °C.

Die bakterizide und fungizide Wirkung der erfindungsgemäßen Formulierungen ist ausgesprochen gut. Von Vorteil für ihre Anwendung im Bereich der maschinellen Instrumentenaufbereitung ist insbesondere ihre inaktivierende Wirkung auf das Hepatitis B-Virus. Das Hepatitis B-Oberflächenantigen wird bereits nach einer Einwirkungszeit von 5 Minuten bei der maschinellen Anwendung zerstört.

Überraschenderweise bilden die erfindungsgemäßen Formulierungen - insbesondere in Form einer Gebrauchslösung - im Gegensatz zum Verhalten der Einzelwirkstoffe selbst bei starker Turbulenz nur eine sehr geringe Schaummenge, wodurch ein Einsatz dieser Wirkstoffe für die maschinelle chemo-thermische Instrumentendesinfektion möglich wird, ohne daß wasserunlösliche Silikonentschäumer oder Fettalkoholalkoxylate eingesetzt werden müssen. Die Kombination aus Hydroxycarbonsäure und Alkansäurealkylester bilden ein synergistisches Entschäumersystem, welches im Vergleich zu üblichen Entschäumern keine Beläge und Ablagerungen bildet.

Ferner verursachen Desinfektionsmittelformulierungen gemäß der vorliegenden Erfindung in ihrer Umgebung keine bzw. nur eine geringe Korrosion. Dies ist naturgemäß insbesondere für die Instrumentendesinfektion von Vorteil, da sowohl die zu desinfizierenden Gegenstände als auch die verwendeten Wasch- und Desinfektionsautomaten im allgemeinen aus Metall gefertigt sind bzw. Metallteile enthalten.

Die Desinfektionsmittelkonzentrate stellen klare, einphasige Mischungen dar, welche in einem Temperaturbereich von 1 bis 50 °C völlig stabil sind. Die wäßrige Gebrauchslösung hingegen, welche vorteilhaft beispielsweise 0,2 bis 2,0 Gew.-% an Konzentrat enthält, kann in einem Temperaturbereich von 20 bis 60 °C eine trübe Emulsion darstellen, die äußerst schaumarm und somit ganz besonders für den maschinellen Einsatzbereich geeignet ist.

Es war in keiner Weise vorhersehbar, daß die Kombination der tensidischen Wirkstoffe mit den erfindungsgemäßen Hilfsstoffen den oben beschriebenen Effekt ermöglichen würde.

Erfindungsgemäß vorteilhafte Komplexbildnerkomponenten sind organische Phosphonatderivate, wie z.B. Hydroxyethylamindimethylenphosphonsäure, Aminotrismethylenphosphonsäure, Hydroxyethan-1,1-diphosphonsäure, 2-Phosphonobutan-tricarbonsäure-1,2,4, Phosphonohydroxyessigsäure u.a.. Es können im Sinne der vorliegenden Erfindung vorteilhaft aber auch andere Komplexbildnerkomponenten, z. B. Nitrilotriessigsäure (NTA), Ethylendiamintetraessigsäure (EDTA), Methylglycindiessigsäure (MGDA), Alanindiessigsäure (ADA) und ihre Natriumsalze, verwendet werden oder Kombinationen dieser Komplexbildnerkomponenten.
Erfindungsgemäß besonders bevorzugt ist die Verwendung einer Kombination von Hydroxyethylamindimethylenphosphonsäure und Methylglycindiessigsäure-Natriumsalz.

Es ist besonders bevorzugt, den Gehalt an dem oder den Komplexbildnerkomponenten in der Desinfektionsmittelformulierung aus dem Bereich zwischen 1 und 50 Gew.-%, insbesondere von 1 bis 10 Gew.-%, bezogen auf das Gesamtgewicht der Formulierung, zu wählen.

Die erfindungsgemäßen Lösungsvermittler werden vorteilhaft aus der Gruppe der Lösungsvermittler gewählt, die üblicherweise in Desinfektionsmitteln enthalten sind. Vorteilhafte Lösungsvermittler im Sinne der vorliegenden Erfindung sind beispielsweise aliphatische Alkohole, Glykole und/oder Polyglykole, wie Dipropylenglykol, Monoethylenglykol, Diethylenglykol, Triethylenglykol, 1,2-Propylenglykol und Copolymerisate von Ethylenoxid und Propylenoxid. Besonders bevorzugt ist die Verwendung von 1,2-Propylenglykol.

Es wird bevorzugt, den Gehalt an einem oder mehreren Lösungsvermittlern zwischen 1 und 50 Gew.-%, insbesondere zwischen 5 und 15 Gew.-%, bezogen auf das Gesamtgewicht der Zubereitungen, zu wählen.

Erfindungsgemäß bevorzugte Hydroxycarbonsäuren sind z. B. Milchsäure, Glykolsäure, Zitronensäure und ihre Natriumsalze. Sie bilden mit den quaternären Ammoniumverbindungen lonenpaare und beeinflussen so deren Schaumeigenschaften maßgeblich. Besonders bevorzugte Hydroxycarbonsäure im Sinne der vorliegenden Erfindung ist Milchsäure.

Es ist besonders bevorzugt, den Gehalt an der oder den Hydroxycarbonsäuren und ihrer Natriumsalze in der Desinfektionsmittelformulierung aus dem Bereich von 1 bis 50 Gew.-%, insbesondere zwischen 1 und 10 Gew.-%, bezogen auf das Gesamtgewicht der Formulierung, zu wählen.

Erfindungsgemäß bevorzugte Alkansäure-Alkylester sind Ester mit einer geradkettigen oder verzweigten Kohlenstoffkette mit 4 bis 10 Kohlenstoffatomen am jeweiligen Ende der Sauerstoffbrücke, z. B. Ethylhexansäure-Ethylhexanolester, Ethylhexansäureisobutylester, Hexansäure-isobutylester, Ethylhexansäurehexanolester und/oder Hexansäure-Ethylhexanolester.

Diese Schaumregulatorkomponenten haben den Vorteil, daß sie in nur sehr geringen Megen eingesetzt werden müssen, um die Schaumeigenschaften der Formulierung maßgeblich zu beeinflussen. Darüberhinaus zeigen sie keine für Entschäumer sonst übliche negativen Eigenschaften wie das Aufziehen auf Instrumenten und den Maschineninnenraum. Besonders bevorzugter Alkansäure-Alkylester im Sinne der vorliegenden Erfindung ist Ethylhexansäure-Ethylhexanolester.

Es ist besonders bevorzugt, den Gehalt an dem oder den Schaumregulatoren in der Desinfektionsmittelformulierung aus dem Bereich von 0,1 bis 5 Gew.-%, insbesondere zwischen 0,1 und 1 Gew.-%, bezogen auf das Gesamtgewicht der Formulierung, zu wählen.

Es ist ferner bevorzugt, den Gehalt an der oder den quaternären Ammoniumverbindung(en) in der Desinfektionsmittelformulierung aus dem Bereich von 0,5 bis 50 Gew.-%, vorzugsweise zwischen 5 und 20 Gew.-%, bezogen auf das Gesamtgewicht der Formulierung, zu wählen. Zu bevorzugen ist hierbei eine Kombination von N-Dodecyl-N,N-dimethyl-ethylbenzyl-ammoniumchlorid und Dioctyldimethylammoniumchlorid.

Die erfindungsgemäßen Desinfektionsmittelformulierungen (in folgenden auch "Desinfektionsmittelkonzentrate" genannt) werden nach Verdünnen mit Wasser - in Form einer sogenannten "Gebrauchslösung" - als Desinfektionsmittel verwendet. Erfindungsgemäß vorteilhaft sind Gebrauchslösungen, welche 0,2 bis 2,0 Gew.-% an Konzentrat enthalten.

Erfindungsgemäß vorteilhaft wird der pH-Wert des Desinfektionsmittelkonzentrates auf Werte zwischen 2 bis 12 und vorzugsweise 5 bis 9 eingestellt. Zur pH-Einstellung können anorganische oder organische Säuren und Basen verwendet werden, wie beispielsweise die o. g. Phosphonsäurederivate und Hydroxycarbonsäuren, welche zudem das Kalkbindevermögen der Anwendungslösung erhöhen.

Ferner können die erfindungsgemäßen Desinfektionsmittelkonzentrate vorteilhaft zusätzlich übliche Korrosionsinhibitoren - wie beispielsweise Benzotriazol - enthalten, insbesondere falls sie bei ihrer Anwendung mit metallischen Oberflächen, insbesondere mit nicht veredelten Stählen oder Werkstoffen aus Buntmetallegierungen, in Berührung kommen können. Es wird bevorzugt, den Gehalt an einem oder mehreren Korrosionsinhibitoren zwischen 0,1 und 5 Gew.-%, bezogen auf das Gesamtgewicht der Konzentrate, zu wählen.

Zusätzlich zu den vorstehend genannten Komponenten können die erfindungsgemäßen Desinfektionsmittelkonzentrate andere übliche Hilfsstoffe, wie z. B. nichtionische Tenside, Komplexbildner, Sequestiermittel, pH-Regulatoren, Lösevermittler und Schaumregulatoren enthalten. Diese dienen der Verbesserung der Gebrauchseigenschaften der Desinfektionsmittelformulierung wie z. B. der Reinigungsleistung, der Wasserenthärtung, der Schaumunterdrückung und der Stabilität.

Zusätzlich zu den vorstehend genannten Komponenten können die erfindungsgemäßen Desinfektionsmittelkonzentrate für derartige Zubereitungen übliche Konservierungsstoffe, Farbstoffe und Duftstoffe enthalten. Diese tragen in der Mehrzahl der Fälle nicht zur desinfizierenden Wirkung bei, sondern dienen der Lagerbarkeit sowie ästhetischen Zwecken. Es ist jedoch auch möglich, solche Komponenten zu verwenden, die eine (konservierende, pflegende usw.) Wirkung entfalten und dabei gleichzeitig für eine bestimmte Farbe und/oder einen angenehmen Duft sorgen.

Die jeweils einzusetzenden Mengen an derartigen Trägerstoffen und Parfüm können in Abhängigkeit von der Art des jeweiligen Produktes vom Fachmann durch einfaches Ausprobieren leicht ermittelt werden.

Die folgenden Beispiele dienen dazu, die Erfindungen zu beschreiben, selbstverständlich ohne daß beabsichtigt ist, die Erfindungen auf diese Beispiele zu beschränken. Alle Prozentanteile sind, soweit nicht anders angegeben, auf das Gesamtgewicht der Zubereitungen bezogen.

### Beispiele:

### Angaben in Gew.-%

| | | |
|---|---|---|
| Beispiel 1: | 1,2-Propylenglykol | 10,0 % |
| | Dioctyldimethylammoniumchlorid | 8,0 % |
| | N-Dodecyl-N,N-dimethyl-ethylbenzylammoniumchlorid | 2,5 % |
| | Hydroxyethylamindimethylenphosphonsäure | 1,2 % |
| | Methylglycindiessigsäure, Natriumsalz | 2,0 % |
| | 2-Ethylhexansäure-2-ethylhexanolester | 0,5 % |
| | Milchsäure | 3,2 % |
| | Korrosioninhibitoren | 0,2 % |
| | Rest: Hilfsstoffe, Wasser | |
| | | |
| Beispiel 2: | 1,2-Propylenglykol | 10,0 % |
| | Dioctyldimethylammoniumchlorid | 8,0 % |
| | N-Dodecyl-N,N-dimethyl-ethylbenzylammoniumchlorid | 2,5 % |
| | Aminotrismethylenphosphonsäure | 1,0 % |
| | Methylglycindiessigsäure, Natriumsalz | 2,0 % |
| | 2-Ethylhexansäure-2-ethylhexanolester | 0,5 % |
| | Glykolsäure | 2,9 % |
| | Korrosioninhibitoren | 0,2 % |
| | Rest: Hilfsstoffe, Wasser | |
| | | |
| Beispiel 3: | 1,2-Propylenglykol | 10,0 % |
| | Dioctyldimethylammoniumchlorid | 8,0 % |
| | N-Dodecyl-N,N-dimethyl-ethylbenzylammoniumchlorid | 2,5 % |
| | Hydroxyethylamindimethylenphosphonsäure | 1,2 % |
| | Methylglycindiessigsäure, Natriumsalz | 2,0 % |
| | 2-Ethylhexansäureisobutylester | 0,5 % |
| | Milchsäure | 3,2 % |
| | Korrosioninhibitoren | 0,2 % |
| | Rest: Hilfsstoffe, Wasser | |

Bei den Beispielen handelt es um Desinfektionsmittelkonzentrate, welche vor ihrer Anwendung mit Wasser verdünnt werden.

Alle Rezepturen sind stabil und bilden in wässriger Verdünnung eine schaumarme Emulsion, welche für einen maschinellen Einsatz geeignet ist.

## Patentansprüche

1. Desinfektionsmittelkonzentrate in Form klarer einphasiger Mischungen für maschinelle chemothermische Instrumentenaufbereitung, welche neben einem Gehalt an mindestens einem Wirkstoff gewählt aus der Gruppe Benzalkoniumchlorid, N-Dodecyl-N,N-dimethyl-ethylbenzylammoniumchlorid, Dioctyldimethylammoniumchlorid, Didecyldimethylammoniumchlorid, Decylisononyldimethylammoniumchlorid und Didecylmethylpolyoxyethylammoniumpropionat
a) 1 bis 50 Gew.-% mindestens einer Komplexbildnerkomponente,
b) 1 bis 50 Gew.-% mindestens eines Lösungsvermittlers,
c) 1 bis 50 Gew.-% mindestens einer Hydroxycarbonsäure und
d) 0,1 bis 5 Gew.-% mindestens eines Alkansäure-Alkylesters als Schaumregulatorkomponente
enthalten, wobei die Prozentangaben jeweils auf das Gesamtgewicht der Formulierungen bezogen sind.

2. Desinfektionsmittelkonzentrate nach Anspruch 1, **dadurch gekennzeichnet, daß** der Gehalt an der oder den quaternären Ammoniumverbindung(en) in der Desinfektionsmittelformulierung aus dem Bereich von 0,5 bis 50 Gew:-%, vorzugsweise zwischen 5 und 20 Gew.-%, bezogen auf das Gesamtgewicht der Formulierung, gewählt wird.

3. Desinfektionsmittelformulierung nach Anspruch 1 oder 2, **dadurch gekennzeichnet, daß** eine Kombination von N-Dodecyl-N,N-dimethyl-ethylbenzyl-ammoniumchlorid und Dioctyldimethylammoniumchlorid gewählt wird.

4. Desinfektionsmittelkonzentrate nach einem der vorstehenden Ansprüche, **dadurch gekennzeichnet, daß** zusätzlich Tenside, Lösevermittler, Korrosionsinhibitoren, Schaumregulatoren, pH-Regulatoren, Komplexbildner, Sequestiermittel und/oder weitere Hilfs-, Zusatz- und/oder Wirkstoffe enthalten sind.

5. Desinfektionsmittelkonzentrate nach einem der vorstehenden Ansprüche, **dadurch gekennzeichnet, daß** es sich um Konzentrate handelt, die vor der Verwendung mit Wasser zu einer Gebrauchslösung verdünnt werden, wobei die Gebrauchslösung 0,2 bis 2,0 Gew.-% an Konzentrat enthält.

6. Desinfektionsmittelkonzentrate nach einem der vorstehenden Ansprüche, **dadurch gekennzeichnet, daß** die Komplexbildnerkomponente(n) aus der Gruppe organische Phosphonatderivate und ihrer Salze gewählt werden, insbesondere Hydroxyethylamindimethylen-phosphonsäure.

7. Desinfektionsmittelkonzentrate nach einem der vorhergehenden Ansprüche, **dadurch gekennzeichnet, daß** die Komplexbildnerkomponente(n) aus der Gruppe der Aminocarbonsäuren und ihrer Salze gewählt werden, insbesondere Methylglycindiessigsäure-Natriumsalz.

8. Desinfektionsmittelkonzentrate nach einem der vorhergehenden Ansprüche, **dadurch gekennzeichnet, daß** der oder die Lösungsvermittlers aus der Gruppe der aliphatischen Alkohole, Glykole und/oder Polyglykole gewählt werden, vorteilhaft aus der Gruppe: Dipropylenglykol, Monoethylenglykol, Diethylenglykol, Triethylenglykol, 1,2-Propylenglykol und Copolymerisaten von Ethylenoxid und Propylenoxid, vorzugsweise 1,2-Propylenglykol.

9. Desinfektionsmittelkonzentrate nach einem der vorhergehenden Ansprüche, **dadurch gekennzeichnet, daß** die Hydroxycarbonsäure oder Hydroxycarbonsäuren eine oder mehrere Carbonsäuregruppen enthalten.

10. Desinfektionsmittelkonzentrate nach einem der vorhergehenden Ansprüche, **dadurch gekennzeichnet, daß** die Hydroxycarbonsäure oder Hydroxycarbonsäuren aus der Gruppe der als Naturstoffe vorkommenden Hydroxycarbonsäuren gewählt werden, insbesondere aus der Gruppe-: Milchsäure, Zitronensäure und Glykolsäure.

11. Desinfektionsmittelkonzentrate nach einem der vorhergehenden Ansprüche, **dadurch gekennzeichnet, daß** der oder die Alkansäure-Alkylester aus der Gruppe der Ester mit einer geradkettigen oder verzweigten Kohlenstoffkette mit 4 bis 10 Kohlenstoffatomen am jeweiligen Ende der Sauerstoffbrücke gewählt werden.

12. Desinfektionsmittelkonzentrate nach einem der vorhergehenden Ansprüche, **dadurch gekennzeichnet, daß** einerseits das Konzentrat in einem Temperaturbereich von 5 bis 50 °C eine klare Mischung darstellt und daß andererseits dessen wässrige Verdünnung, welche 0,2 bis 2,0 Gew.-% an Konzentrat enthält, in einem Temperaturbereich von 20 bis 60° C eine trübe, mehrphasige Emulsion darstellen kann, welche sich durch ihre schwachschäumende Eigenschaft auszeichnet.

13. Verwendung von Gebrauchslösungen nach Anspruch 5 als Desinfektionsmittel für medizinisch-chirurgische Instrumente in Wasch- und/oder Desinfektionsautomaten.

14. Verwendung von Gebrauchslösungen nach Anspruch 5 als Desinfektionsmittel für die chemo-thermische Aufbereitung flexibler Endoskope.

## Claims

1. Concentrated disinfecting compositions in the form of clear one-phase mixtures for mechanical chemical-thermal care for instruments, which, in addition to having a content of at least one active compound chosen from the group of benzalkonium chloride, N-dodecyl-N,N-dimethyl-ethylbenzylammonium chloride, dioctyldimethylammonium chloride, didecyldimethylammonium chloride, decylisononyldimethylammonium chloride and didecylmethylpolyoxyethylammonium propionate, comprise
a) 1 to 50 wt.% of at least one complexing agent component,
b) 1 to 50 wt.% of at least one solubilizing agent,
c) 1 to 50 wt.% of at least one hydroxycarboxylic acid and
d) 0.1 to 5 wt.% of at least one alkanoic acid alkyl ester as a foam regulator component
the percentage data in each case being based on the total weight of the formulations.

2. Concentrated disinfecting compositions according to claim 1, **characterized in that** the content of the quaternary ammonium compound(s) in the disinfecting composition formulation is chosen from the range of from 0.5 to 50 wt.%, preferably between 5 and 20 wt.%, based on the total weight of the formulation.

3. Disinfecting composition formulation according to claim 1 or 2, **characterized in that** a combination of N-dodecyl-N,N-dimethyl-ethylbenzyl-ammonium chloride and dioctyldimethylammonium chloride is chosen.

4. Concentrated disinfecting compositions according to one of the preceding claims, **characterized in that** they additionally comprise surfactants, solubilizing agents, corrosion inhibitors, foam regulators, pH regulators, complexing agents, sequestering agents and/or further auxiliary substances, additives and/or active compounds.

5. Concentrated disinfecting compositions according to one of the preceding claims, **characterized in that** they are concentrates which before use are diluted with water to give a solution for use, the solution for use containing 0.2 to 2.0 wt.% of concentrate.

6. Concentrated disinfecting compositions according to one of the preceding claims, **characterized in that** the complexing agent component(s) are chosen from the group of organic phosphonate derivatives and their salts, in particular hydroxyethylaminedimethylene-phosphonic acid.

7. Concentrated disinfecting compositions according to one of the preceding claims, **characterized in that** the complexing agent component(s) are chosen from the group of aminocarboxylic acids and their salts, in particular methylglycinediacetic acid sodium salt.

8. Concentrated disinfecting compositions according to one of the preceding claims, **characterized in that** the solubilizing agent(s) are chosen from the group of aliphatic alcohols, glycols and/or polyglycols, advantageously from the group: dipropylene glycol, monoethylene glycol, diethylene glycol, triethylene glycol, 1,2-propylene glycol and copolymers of ethylene oxide and propylene oxide, preferably 1,2-propylene glycol.

9. Concentrated disinfecting compositions according to one of the preceding claims, **characterized in that** the hydroxycarboxylic acid or hydroxycarboxylic acids contain one or more carboxylic acid groups.

10. Concentrated disinfecting compositions according to one of the preceding claims, **characterized in that** the hydroxycarboxylic acid or hydroxycarboxylic acids are chosen from the group of hydroxycarboxylic acids which occur as natural substances, in particular from the group: lactic acid, citric acid and glycolic acid.

11. Concentrated disinfecting compositions according to one of the preceding claims, **characterized in that** the alkanoic acid alkyl ester(s) are chosen from the group of esters with a straight-chain or branched carbon chain having 4 to 10 carbon atoms on the particular end of the oxygen bridge.

12. Concentrated disinfecting compositions according to one of the preceding claims, **characterized in that** on the one hand the concentrate is a clear mixture in a temperature range of from 5 to 50 °C, and that on the other hand the aqueous dilution thereof which contains 0.2 to 2.0 wt.% of concentrate can be a cloudy, multi-phase emulsion, which is distinguished by its low-foaming property, in a temperature range of from 20 to 60 °C.

13. Use of solutions for use according to claim 5 as disinfecting compositions for medical-surgical instruments in washing and/or disinfecting machines.

14. Use of solutions for use according to claim 5 as disinfecting compositions for chemical-thermal care of flexible endoscopes.

## Revendications

1. Concentrés d'agent désinfectant sous forme de mélanges mono-phasiques limpides conçus pour la préparation chimiothermique d'instruments en machine, qui contiennent, en plus d'au moins une substance active choisie dans l'ensemble formé par le chlorure de benzalkonium, le chlorure de N-dodécyl-N,N-diméthyl-éthylbenzyl-ammonium, le chlorure de dioctyl-diméthyl-ammonium, le chlorure de didécyl-diméthyl-ammonium, le chlorure de décyl-isononyl-diméthyl-ammonium et le propionate de didécyl-méthyl-polyoxyéthyl-ammonium,
a) 1 à 50 % en poids d'au moins un composant complexant,
b) 1 à 50 % en poids d'au moins un tiers-solvant,
c) 1 à 50 % en poids d'au moins un acide hydroxy-carboxylique,
d) et 0,1 à 5 % en poids d'au moins un ester alkylique d'acide alcanoïque, en tant que composant régulateur du moussage,
ces pourcentages étant rapportés au poids total de la formulation.

2. Concentrés d'agent désinfectant, conformes à la revendication 1, **caractérisés en ce que** la teneur de la formulation d'agent désinfectant en le ou les composé(s) d'ammonium quaternaire est choisie dans l'intervalle allant de 0,5 à 50 % et de préférence de 5 à 20 %, en poids rapporté au poids total de la formulation.

3. Concentrés d'agent désinfectant, conformes à la revendication 1 ou 2, **caractérisés en ce qu'**on a choisi d'utiliser une combinaison de chlorure de N-dodécyl-N,N-diméthyl-éthylbenzyl-ammonium et de chlorure de dioctyl-diméthyl-ammonium.

4. Concentrés d'agent désinfectant, conformes à l'une des revendications précédentes, **caractérisés en ce qu'**ils contiennent en outre des tensioactifs, des tiers-solvants, des inhibiteurs de corrosion, des agents de régulation du moussage, des agents d'ajustement du pH, des agents complexants, des agents séquestrants, et/ou d'autres auxiliaires, adjuvants et/ou substances actives.

5. Concentrés d'agent désinfectant, conformes à l'une des revendications précédentes, **caractérisés en ce qu'**il s'agit de concentrés qu'on dilue avec de l'eau, avant de les utiliser, pour en faire des solutions prêtes à l'emploi qui contiennent de 0,2 à 2,0 % en poids de concentré.

6. Concentrés d'agent désinfectant, conformes à l'une des revendications précédentes, **caractérisés en ce que** le ou les composant(s) complexant(s) est ou sont choisi(s) parmi les dérivés organiques de type phosphonate et leurs sels, en particulier l'acide hydroxyéthylamine-diméthylènephosphonique.

7. Concentrés d'agent désinfectant, conformes à l'une des revendications précédentes, **caractérisés en ce que** le ou les composant(s) complexant(s) est ou sont choisi(s) parmi les acides aminocarboxyliques et leurs sels, en particulier le sel de sodium de l'acide méthyl-glycine-diacétique.

8. Concentrés d'agent désinfectant, conformes à l'une des revendications précédentes, **caractérisés en ce que** le ou les tiers-solvant(s) est ou sont choisi(s) parmi les alcools aliphatiques, glycols et/ou polyglycols, et de préférence parmi les dipropylèneglycol, monoéthylèneglycol, diéthylèneglycol, triéthylèneglycol et 1,2-propylèneglycol et les copolymères d'oxyde d'éthylène et d'oxyde de propylène, surtout le 1,2-propylèneglycol.

9. Concentrés d'agent désinfectant, conformes à l'une des revendications précédentes, **caractérisés en ce que** le ou les acide(s) hydroxy-carboxylique(s) comporte(nt) un ou plusieurs groupe(s) carboxyle.

10. Concentrés d'agent désinfectant, conformes à l'une des revendications précédentes, **caractérisés en ce que** le ou les acide(s) hydroxy-carboxylique(s) est ou sont choisi(s) parmi les acides hydroxy-carboxyliques classés comme produits naturels, et en particulier parmi l'acide lactique, l'acide citrique et l'acide glycolique.

11. Concentrés d'agent désinfectant, conformes à l'une des revendications précédentes, **caractérisés en ce que** le ou les ester(s) alkylique(s) d'acide alcanoïque est ou sont choisi(s) parmi les esters qui comportent, de chaque côté du groupe pontant acide, une chaîne carbonée, linéaire ou ramifiée, de 4 à 10 atomes de carbone.

12. Concentrés d'agent désinfectant, conformes à l'une des revendications précédentes, **caractérisés en ce que**, d'une part, le concentré se présente comme un mélange limpide dans l'intervalle de température allant de 5 à 50 °C, et **en ce que**, d'autre part, une dilution du concentré dans de l'eau, contenant de 0,2 à 20 % en poids de concentré, se présente, dans l'intervalle de température allant de 20 à 60 °C, comme une émulsion poly-phasique trouble, qui se signale par son faible pouvoir moussant.

13. Utilisation des solutions prêtes à l'emploi, définies dans la revendication 5, en tant qu'agents désinfectants pour instruments médico-chirurgicaux dans des appareils automatiques de lavage et/ou de désinfection.

14. Utilisation des solutions prêtes à l'emploi, définies dans la revendication 5, en tant qu'agents désinfectants pour la préparation chimiothermique d'endoscopes flexibles.
